# EUROPEAN PATENT SPECIFICATION

(11) **EP 1 234 558 B1**
(45) Date of publication and mention of the grant of the patent: **01.03.2006**
(21) Application number: 02076870.1
(22) Date of filing: 26.06.1998
(51) Int. Cl.: A61F 2/46

(54) **Modular hip trial for long stems**
Modulare Hüftprobeprothese für lange Schäfte
Prothèse d'essai modulaire de la hanche pour tiges longues

(30) Priority: 27.06.1997 US 884588
(43) Date of publication of application: 28.08.2002
(62) Divisional of application: 98305075.8
(73) Proprietor: DePuy Products, Inc., Warsaw, IN 46581 (US)
(72) Inventor: Ro, Gloria, North Quincy, MA 02170 (US)
(74) Representative: Fisher, Adrian John

(56) References cited:
- EP-A- 0 163 121
- WO-A-96/00539
- FR-A- 2 660 857
- US-A- 4 963 155
- US-A- 5 032 130
- US-A- 5 100 407
- US-A- 5 507 817
- US-A- 5 569 263

## Description

### RELATED APPLICATION

The present application is divided from European patent application No. 98 305 075.8 (publication No. 0 887 053) filed 26th June 1998.

### FIELD OF THE INVENTION

The present invention relates to a device used in hip arthroplasty, such as a trial for determining the required dimensions of a prosthetic femoral component, and more particularly to a trial for a calcar stem.

### BACKGROUND OF THE INVENTION

A successful hip replacement or arthroplasty procedure results, in part, from selection of prosthetic joint components that are dimensioned and positioned to closely approximate or replicate the geometry and functional characteristics of a natural, healthy hip joint. Typically, the component selection process includes a pre-operative analysis of joint images. However, it has been discovered that a valuable adjunct to image analysis is the temporary fixation of one or more provisional components to a bone or bones of interest at a stage of the arthroplasty procedure prior to permanent fixation of the prosthetic joint. The provisional components are intended to mimic certain aspects of the permanent prosthetic joint in order for a surgeon to validate measurements and to test or "try-out" several different possible component sizes and configurations. Hence, provisional components are aptly known as "trials."

In a known procedure, a trial for a femoral component is used in the following manner. The proximal end of a femur is resectioned and the medullary canal of the femur is reamed. A broach is inserted into the resected proximal end of the femur to provide a cavity within the bone dimensioned and contoured to receive a femoral stem. However, prior to removing the broach, a trial neck or trunnion and trial head can be secured to the broach to simulate a complete femoral stem. Normally, several neck and head trials of varying lengths and geometries are successively joined to the broach in an attempt to determine an appropriate neck length and overall femoral stem length. Once these lengths have been determined, the trial neck and head are removed from the broach and the broach is removed from the femur. Subsequently, a femoral stem of the appropriate length is selected for insertion into the cavity defined by the broach using techniques known to those skilled in the art.

Other techniques require that the broach be removed from the medullary canal to allow a trial having a stem portion to be used, in addition to a trial head and neck. For example, United States Patent No. 5,100,407 discloses a system including a group of variously sized trial neck/body portions and a group of differing length trial stem portions which are mixed and matched to create a suitable trial. However, repetitive removal and insertion of successions of trial stems accompanied by successive assembly and disassembly with respect to the body can consume a lengthy and costly period of time.

Another known trial includes a stem to which a collar is secured at successive points along the length of the trial until an appropriate neck length and stem length have been ascertained. Undesirably, this type of trial induces measurement inaccuracies resulting from stem movement as the collar is repeatedly engaged with and disengaged from the stem. Additionally, as the collar is moved toward the distal end of the stem, less and less of the stem is disposed within the medullary canal, causing the trial to become increasingly unstable and rendering accurate measurements very difficult to achieve.

In addition to other deficiencies of known trials, it is believed that a trial system consisting of numerous parts that must be selected and mated in various combinations, possibly many times, is cumbersome, unnecessarily complex and a waste of valuable time.

In US patent No. 5601567 these is made known a multi-piece trial femoral kit comprising interengagable proximal and distal stem positions.

### SUMMARY OF THE INVENTION

The present invention overcomes the disadvantages of known trials by providing a unified assembly that facilitates very accurate measurements in a convenient, easy to use manner. The trial does not require repeated assembly and disassembly, and it is uniquely able to provide a geometry that closely approximates a broached cavity, regardless of the height of the trial.

According to the present invention these is provided a hip replacement system according to claim 1.

Further preferred features of the invention are set out in the subordinate claims.

### BRIEF DESCRIPTION OF THE DRAWINGS

A more complete understanding of the present invention and the attendant advantages and features thereof will be more readily understood by reference to the following detailed description when it is considered in conjunction with the accompanying drawings, wherein:
FIG. 1 is an exploded view of the trial in accordance with the invention that illustrates a body portion and a stem portion;
FIG. 2 is a perspective view of the stem portion of the trial shown in FIG. 1;
FIG. 3 is a top view of the body portion illustrated in FIG. 1;
FIG. 4 is a sectional view of a trial showing a locking mechanism in an engaged position;
FIG. 5 is a sectional view of a trial showing the locking mechanism in a disengaged position;
FIG. 6 is a side view of the trial of FIG. 1 in an assembled configuration;
FIG. 7 is a side view of the trial in accordance with the invention inserted into a femur at a first body height;
FIG. 8 illustrates a trial in accordance with the invention inserted into a femur at a second body height;
FIG. 9 is a perspective view of an inserter/extractor tool;
FIG. 10 illustrates the tool of FIG. 9 engaged with a trial for insertion of the trial into a femur;
FIG. 11 illustrates the insertion/extraction tool in an extraction position;
FIG. 12 is an exploded view of an embodiment of a trial having a modular stem portion;
FIG. 13 depicts the trial of FIG. 12 in an assembled state; and
FIG. 14 is a partial cut-away view of a connection between a first stem portion and a second stem portion.

### DETAILED DESCRIPTION OF THE INVENTION

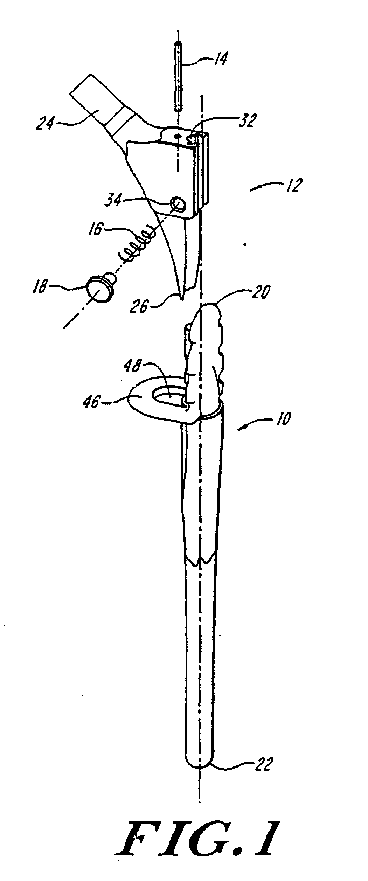

Referring to FIG. 1, a trial in accordance with the invention is shown in an exploded view to show a stem 10, a body 12, a pin 14, a spring 16, and a push-button 18. The stem 10 has a proximal end 20 and a distal end 22, and the body 12 has a proximal end defining a trunnion 24 and a distal end 26. The body 12 is engagable with the stem 10 near the proximal end of the stem so as to be slidable with respect to the stem a predetermined distance between the proximal end and the distal end of the stem.

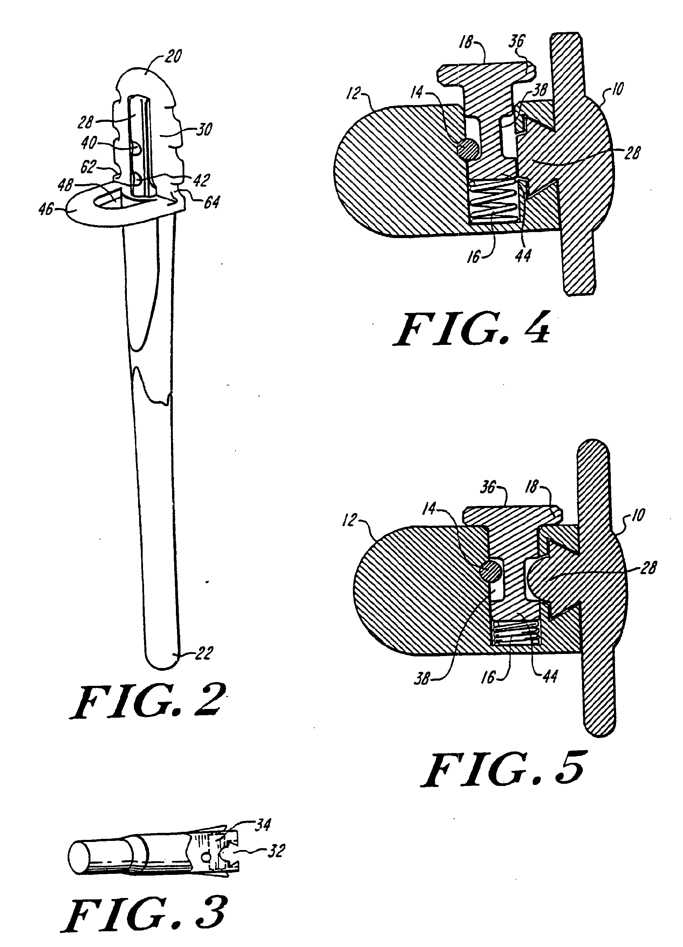

A guide or track can be associated with either or both of the body 12 and the stem 10 to guide movement of body with respect to the stem. For example, as shown in FIG. 2, the stem 10 includes a track 28 protruding from a first face 30 of the stem and extending a predetermined distance between the proximal end 20 and the distal end 22 of the stem. As shown in FIG. 1, the body 10 includes a notch 32 for receiving the track 28. However, in other embodiments, the body 12 includes a raised portion that is engagable with a track that is recessed within the stem. Regardless of its configuration, the complementary guide/track/notch of the body and stem serve to limit movement of the body 12 along a predetermined path, such as longitudinal movement, as well as to inhibit undesired movements such as twisting or lateral displacement.

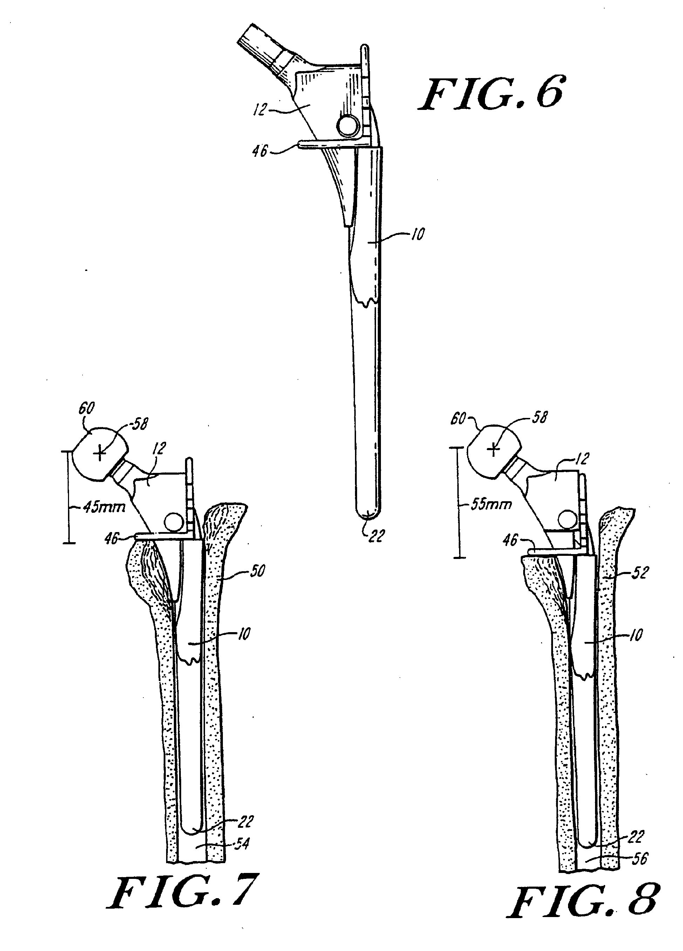

Additionally, a locking mechanism can be provided for inhibiting movement of the body 12 with respect to the stem 10. As shown in FIGS. 1 and 3, the body 12 can include a channel 34 that is transverse to the notch 32 and which is adapted to receive an elongate portion of the button 18 therein. The button 18 is movable within the channel from a first position, wherein a portion of the button contacts and engages a portion of the track 28 of the stem, to a second position wherein the button is disengaged from the track. As shown in FIG. 4, the button 18 is biased to the first position by the spring 16. FIG. 5 illustrates the button 18 in the second position. The button 18 includes an expanded head portion 36 that engages the body 12 to limit insertion depth of the button 18 into the body. The button 18 also includes a cut-out portion 38 into which the pin 14 and a portion of the track 28 are received. It will be noted most clearly in FIG. 2 that the track includes first and second transverse grooves 40 and 42. When the body 12 and the stem 10 are caused to slide with respect to each other, the cut-out portion 38 rides over/along the track 28 until a groove 40, 42 is reached, whereupon an end portion 44 of the button is biased into the groove 40, 42. The end portion 44 is released from the groove 40, 42 by pushing the button 18 into the body 12 with enough force to overcome the bias force of the spring 16.

Referring again to FIGS. 1 and 2, a collar 46 extends radially outward from the stem. The collar 16 surrounds a portion of the body 12, as shown in FIG. 6, and it is dimensioned to be disposed on a resectioned bone surface as-shown in FIGS. 7 and 8. The collar 46 and the distal end of the stem 22 are a fixed distance apart. The collar defines an aperture 48 having curves to complement the shape of the body 12 and into which the body is received.

Turning now to FIGS. 7 and 8, use of the calcar trial is illustrated with respect to resectioned femurs 50 and 52 respectively. Once the femur has been prepared to receive the trial, the distal end 22 of the trial is inserted into the medullary canal 54, 56. It should be noted that in both FIGS. 7 and 8 the full length of the stem 10 from the collar 46 to the distal end of the stem 22 is inserted into the medullary canal. This enables a surgeon to verify that the medullary canal has been reamed to a sufficient depth and width to accommodate a replacement hip stem. The surgeon then slides the body 12 with respect to the stem as required to adjust the head height or distance between a reference point 58 on a head 60 affixed to the body 12 and the collar 46 to determine a prosthetic hip stem length. In FIG. 7, the body 12 is positioned with respect to the stem 10 at a head height of 45mm, whereas in FIG. 8 the head height is 55mm. The trial is then removed from the medullary canal and a prosthetic hip stem having the determined length is selected from a group of hip stems. The selected hip stem is cemented into the medullary canal.

It should be noted in these illustrations that the proximal portion of the trial underneath the collar is wider than an intermediate portion of the stem or its distal end 22 to ensure a tight fit of the trial within the femur. The trial is slightly larger than an actual replacement stem in the proximal section below the collar to allow the trial to fill the medullary canal which has been reamed to be slightly larger than an actual replacement stem (to leave room for bone cement to surround the replacement stem). Also, the stem 10 and the body 12 are configured so that a diameter of the trial at a point between the collar and the distal end of the stem increases as the body is moved toward the distal end of the stem and decreases as the body is moved away from the distal end of the stem. Thus, regardless of where a resection cut is made, the dimensions and shape of the trial correspond to the dimensions and shape of the broached medullary canal.

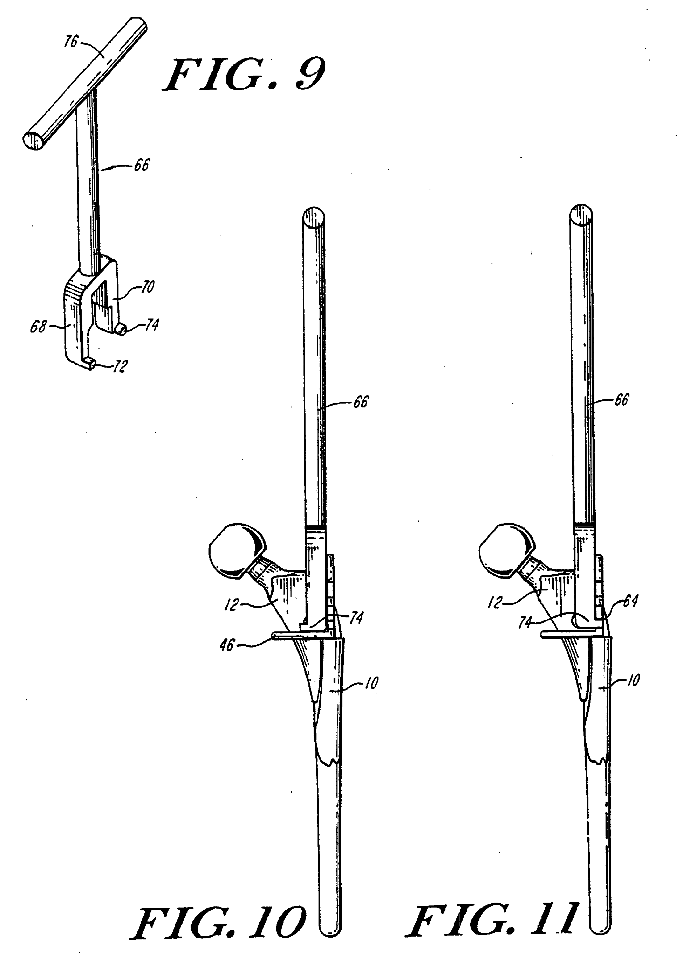

As the trial is usually seated within the medullary canal very snugly, the trial in accordance with the invention further includes features that are of use when inserting the trial into or extracting the trial from the medullary canal. For example, referring to FIG. 2, the proximal end of the stem includes a tool engagement structure, such as a pair of opposed notches 62 and 64 on the stem. A tool 66, shown in FIG. 10, includes a first fork portion or furcation 68 and a second furcation 70 for engaging the opposed notches 62 and 63. The space between the furcations corresponds to the shape of the body 12 to allow the tool to snugly interfit with the body. This ensures that the tool remains axially aligned with the body 12 and the stem 10. Each furcation 68 and 70 can include angled end portions or tines 72 and 74, respectively. A handle 76 provides an easily graspable structure for pulling or pushing tool 66 as well as a suitable surface for mallet striking. Because the notches 62, 64 are similar to the notches of a replacement stem, used to orient cerclage cables, the same tool 66 can be used to insert and extract both the trial and the replacement stem.

FIG. 10 shows the tool 66 positioned with respect to the trial for insertion of the trial into a medullary canal, wherein the tines 72, 74 are not engaged with the notches 62, 64, but rest directly upon the collar 46 on opposite sides of the body 12.

FIG. 11 shows the tool 66 positioned with respect to the trial for extraction of the trial from a medullary canal, wherein the tines 72, 74 are engaged with the notches 62, 64. The forked tool thus allows an even and distributed force to be applied to the stem 10 during both insertion and extraction.

Yet another problem with trials, particularly trials for long hip stems, it that a long trial must account for the curvature or bow of the femur. Accordingly, the present invention further provides a modular stem that, depending on the configuration of a distal stem portion, can lengthen the trial stem and provide required curvature for appropriate femoral implantation.

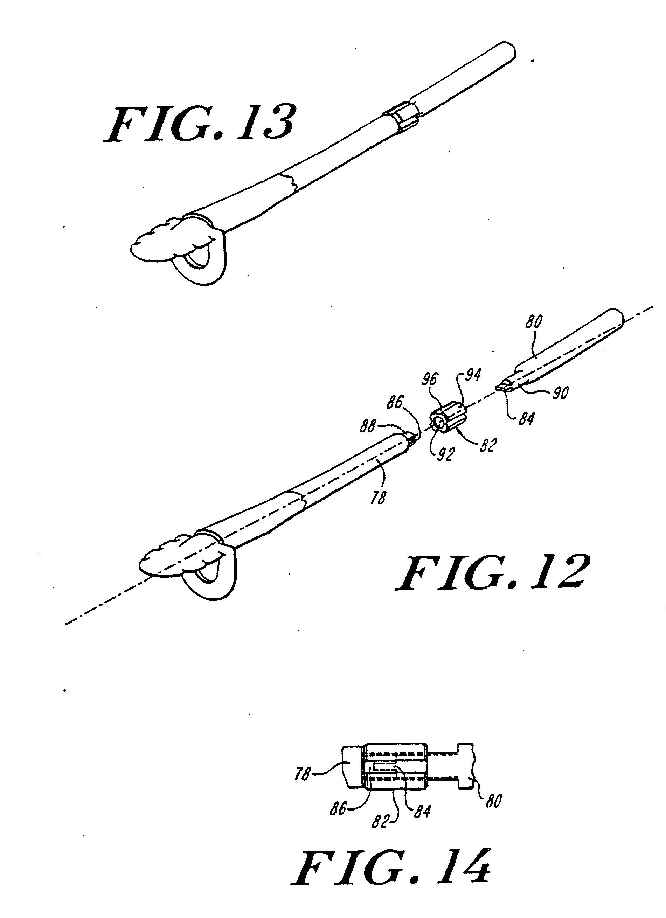

For example, FIG. 12 is an exploded view of an embodiment of a trial having a proximal stem portion 78, a distal stem portion 80, and a connecting element 82. The proximal stem portion 78 can include one or more of the features described above with respect to engagement of a body 12 or with respect to a tool 66. Alternatively, the proximal stem portion 78 can include none of the above features, but rather features found in trials known in the art.

In the illustrated embodiment, the proximal stem portion 78 is about the same length as the stem portion 10 illustrated in FIGS. 1-11 and is therefore usable as described above without the addition of the connecting element 82 or the distal stem portion 82. However, when the distal stem portion 80 is joined to the proximal stem portion 78 the total length of the trial is equivalent to that of a traditional single-piece, long-stem trial. In other embodiments, the proximal stem portion 78 is longer or shorter than the stem portion 10, and the distal stem portion 80 is correspondingly shorter or longer than that illustrated in FIG. 12.

Although the distal stem portion 80 can be straight or coaxial with the proximal stem portion 78, in the illustrated embodiment it is curved or includes an angulation so that some or all of the distal stem portion is not coaxial with the proximal stem portion. The curvature or the angulation can be provided by the shape of the distal stem portion and/or by joining the stem portions together at an angle. Collectively, curvature or angulation of the stem or stems is referred to as "curved."

Continuing to refer to FIG. 12, the distal stem portion 82 is selectively, reversibly attachable to the proximal stem portion 78 to cause the trial to have a curve in a first or a second opposing direction. Thus, the trial can be used for either the right or the left femur without requiring an additional part. In the illustrated embodiment, structures that facilitate reversibility include a tang 84 on the distal stem portion 80 that is receivable within a slot 86 defined by the proximal stem portion 78. The tang 84 and the slot 86 ensure that the distal and proximal stem portions are mated at a precise orientation, and that the orientation cannot be inadvertently changed. For example, were the stem portions to be joined simply by friction fitting a cylindrical element into a cylindrical bore, or by a threaded connection, precise orientation could not be assured.

The slot 86 and tang 84 can be located on reduced diameter portions 88 and 90 of the respective proximal stem portion 78 and the distal stem portion 80. The reduced diameter portions 88 and 90 can be threaded and receivable within a threaded bore 92 of the connecting element 82. The connecting element 82 can include texturing such as alternating elongate ridges 94 and depressions 96 to enable the connecting element to be grasped by hand or with a tool and rotated with respect to the stem portion or portions to create a threaded engagement between the stem portions. Thus, the connecting element locks the stem portions together. However, other embodiments are contemplated that allow the stem portions to be joined in one of two or more precise orientations and be held together without a separate connecting element.

FIG. 13 depicts the trial of FIG. 12 in an assembled state. It should be noted that the connecting element 82 has a maximum diameter that approximates that of the stem portions where they join the connecting element.

FIG. 14 is a partial cut-away view of a connection between the proximal stem portion 78 and the distal stem portion 80 which clearly shows the engagement of the tang 84 with the slot 86. Although the illustrated tang and slot provide an excellent reversible connection, other structures for joining the stem portions are contemplated and the particular connection is not a limitation of the invention.

Although the invention has been shown and described with respect to exemplary embodiments thereof, various other changes, omissions and additions in form and detail thereof may be made without departing from the scope of the invention as defined by the appended claims.

## Claims

1. A hip replacement system comprising:
a trial for a prosthetic femoral implant including a proximal stem portion (78) having a proximal end and a distal end; and **characterised by** further comprising a plurality of distal stem portions (80), wherein the distal stem portions are securable to the distal end of the proximal stem portion; a body (12) slidably engagable with the proximal stem portion, wherein the body is slidable a predetermined distance between the proximal end and the distal end of the proximal stem portion; and
a vertical flange at the proximal stem portion that is wider than the body and which includes a pair of opposed notches (62,64) on opposite sides of the body.

2. The hip replacement system of claim 1, further comprising a connecting element (82) having a first end and a second end, the connecting element being interposable between a proximal end of each distal stem portion and the distal end of the proximal stem portion, wherein the connecting element is releasably securable at the first end to the distal end of the proximal stem portion and releasably securable at the second end to the proximal end of each distal stem portion.

3. The hip replacement system of claim 1 or 2, wherein each distal stem portion includes a tang at its proximal end and wherein the distal end of the proximal stem portion defines a slot (86) for receiving the tang.

4. The hip replacement system of claim 1, 2 or 3, wherein the distal stem portions are curved.

5. The hip replacement system of claim 4, wherein each distal stem portion is engagable with the proximal stem portion in a first orientation to cause the distal stem portion to curve in a first direction with respect to the proximal stem portion, and wherein each distal stem portion is engagable with the proximal stem portion in a second orientation to cause the distal stem portion to curve in a second direction with respect to the proximal stem portion.

6. The hip replacement system of claim 5, wherein the first direction is opposite from the second direction.

7. The hip replacement system of claim 2 or any one of claims 3 to 6 when appended to claim 2, wherein the connecting element includes a threaded bore (92) and wherein the distal end of the proximal stem portion and the proximal end of the distal stem portion are threaded and insertable into the bore of the connecting element.

8. The hip replacement system of claim 2, any one of claims 3 to 6 when appended to claim 2, or claim 7, wherein an outer surface portion of the connecting element is textured (94,96).

9. The hip replacement system of claim 8, wherein the outer surface of the connecting element includes a plurality of ridges (94) and depressions (96).

10. The hip replacement system of claim 1, further comprising a connecting element binding the distal end of the proximal stem portion to the proximal end of the distal stem portion.

## Patentansprüche

1. Hüftersatzsystem, umfassend:
eine Testvorrichtung für ein prothetisches femorales Implantat, umfassend einen proximalen Schaftabschnitt (78) mit einem proximalen und einem distalen Ende; und
mehrere distale Schaftabschnitte (80), wobei die distalen Schaftabschnitte am distalen Ende des proximalen Schaftabschnitts befestigbar sind;
**dadurch gekennzeichnet, daß** es des weiteren einen Körper (12), der verschiebbar mit dem proximalen Schaftabschnitt in Eingriff gebracht werden kann, wobei der Körper über eine vorbestimmte Distanz zwischen dem proximalen Ende und dem distalen Ende des proximalen Schaftabschnitts verschiebbar ist; und
einen vertikalen Flansch am proximalen Schaftabschnitt umfaßt, der breiter ist als der Körper und der ein Paar sich gegenüberliegender Aussparungen (62, 64) auf gegenüberliegenden Seiten des Körpers umfaßt.

2. Hüftersatzsystem nach Anspruch 1, das des weiteren ein Verbindungselement (82) mit einem ersten und einem zweiten Ende umfaßt, wobei das Verbindungselement zwischen einem proximalen Ende jedes distalen Schaftabschnitts und dem distalen Ende des proximalen Schaftabschnitts einfügbar ist, wobei das Verbindungselement am ersten Ende lösbar am distalen Ende des proximalen Schaftabschnitt und am zweiten Ende lösbar am proximalen Ende jedes distalen Schaftabschnitts befestigbar ist.

3. Hüftersatzsystem nach Anspruch 1 oder 2, wobei jeder distale Schaftabschnitt an seinem proximalen Ende einen Zapfen aufweist und am proximalen Schaftabschnitt ein Schlitz (86) zur Aufnahme des Zapfens definiert ist.

4. Hüftersatzsystem nach Anspruch 1, 2 oder 3, wobei die distalen Schaftabschnitte gekrümmt sind.

5. Hüftersatzsystem nach Anspruch 4, wobei jeder distale Schaftabschnitt mit dem proximalen Schaftabschnitt in einer ersten Orientierung in Eingriff gebracht werden kann, um zu bewirken, daß sich der distale Schaftabschnitt in einer ersten Richtung im Verhältnis zum proximalen Schaftabschnitt krümmt, und wobei jeder distale Schaftabschnitt mit dem proximalen Schaftabschnitt in einer zweiten Orientierung in Eingriff gebracht werden kann, um zu bewirken, daß sich der distale Schaftabschnitt in einer zweiten Richtung im Verhältnis zum proximalen Schaftabschnitt krümmt.

6. Hüftersatzsystem nach Anspruch 5, wobei die erste Richtung zur zweiten Richtung entgegengesetzt ist.

7. Hüftersatzsystem nach Anspruch 2 oder nach einem der Ansprüche 3 bis 6, wenn angefügt an den Anspruch 2, wobei das Verbindungselement eine mit einem Gewinde versehene Bohrung (92) umfaßt und das distale Ende des proximalen Schaftabschnitts und das proximale Ende des distalen Schaftabschnitts mit einem Gewinde versehen sind und in die Bohrung des Verbindungselement eingefügt werden können.

8. Hüftersatzsystem nach Anspruch 2, nach einem der Ansprüche 3 bis 6, wenn angefügt an den Anspruch 2, oder nach Anspruch 7, wobei ein äußerer Oberflächenabschnitt des Verbindungselements strukturiert ist (94, 96).

9. Hüftersatzsystem nach Anspruch 8, wobei die äußere Oberfläche des Verbindungselements mehrere Rippen (94) und Vertiefungen (96) umfaßt.

10. Hüftersatzsystem nach Anspruch 1, das des weiteren ein Verbindungselement umfaßt, das das distale Ende des proximalen Schaftabschnitts mit dem proximalen Ende des distalen Schaftabschnitts verbindet.

## Revendications

1. Système de remplacement de la hanche comprenant :
un essai pour un implant fémoral prothétique comprenant une partie formant tige proximale (78) ayant une extrémité proximale et une extrémité distale ; et
une pluralité de parties formant tige distale (80), dans lequel les parties formant tige distale peuvent être fixées à l'extrémité distale de la partie formant tige proximale;
**caractérisé en ce qu'**il comprend en outre :
un corps (12) pouvant s'engager de manière coulissante avec la partie formant tige proximale, dans lequel le corps peut coulisser sur une distance prédéterminée entre l'extrémité proximale et l'extrémité distale de la partie formant tige proximale; et
une bride verticale au niveau de la partie formant tige proximale qui est plus large que le corps et qui comprend une paire d'encoches opposées sur les bords opposés du corps.

2. Système de remplacement de la hanche selon la revendication 1, comprenant en outre un élément d'assemblage (82) ayant une première extrémité et une seconde extrémité, l'élément d'assemblage pouvant être interposé entre une extrémité proximale de chaque partie formant tige distale et l'extrémité distale de la partie formant tige proximale, dans lequel l'élément d'assemblage peut être fixé de façon amovible au niveau de la première extrémité à l'extrémité distale de la partie formant tige proximale et fixé de façon amovible au niveau de la seconde extrémité à l'extrémité proximale de chaque partie formant tige distale.

3. Système de remplacement de la hanche selon la revendication 1 ou 2, dans lequel chaque partie formant tige distale comprend un tenon au niveau de son extrémité proximale et dans lequel l'extrémité distale de la partie formant tige proximale définit une fente (86) permettant de recevoir le tenon.

4. Système de remplacement de la hanche selon la revendication 1, 2 ou 3, dans lequel les parties formant tige distale sont incurvées.

5. Système de remplacement de la hanche selon la revendication 4, dans lequel chaque partie formant tige distale peut s'engager avec la partie formant tige proximale dans une première direction pour provoquer la courbure de la partie formant tige distale dans une première direction par rapport à la partie formant tige proximale, et dans lequel chaque partie formant tige distale peut s'engager avec la partie formant tige proximale dans une seconde direction pour provoquer la courbure de la partie formant tige distale dans une seconde direction par rapport à la partie formant tige proximale.

6. Système de remplacement de la hanche selon la revendication 5, dans lequel la première direction est à l'opposé de la seconde direction.

7. Système de remplacement de la hanche selon la revendication 2, ou selon l'une quelconque des revendications 3 à 6 lorsqu'elles dépendent de la revendication 2, dans lequel l'élément d'assemblage comprend un trou alésé fileté (92) et dans lequel l'extrémité distale de la partie formant tige proximale et l'extrémité proximale de la partie formant tige distale sont filetées et peuvent être insérées dans le trou alésé de l'élément d'assemblage.

8. Système de remplacement de la hanche selon la revendication 2, selon l'une quelconque des revendications 3 à 6 lorsqu'elles dépendent de la revendication 2, ou selon la revendication 7, dans lequel une partie formant surface externe de l'élément d'assemblage est texturé (94, 96).

9. Système de remplacement de la hanche selon la revendication 8, dans lequel la surface externe de l'élément d'assemblage comprend une pluralité de crêtes (94) et de dépressions (96).

10. Système de remplacement de la hanche selon la revendication 1, comprenant en outre un élément d'assemblage reliant l'extrémité distale de la partie formant tige proximale à l'extrémité proximale de la partie formant tige distale.
